# EUROPÄISCHE PATENTSCHRIFT

(11) **EP 4 231 946 B1**
(45) Veröffentlichungstag und Bekanntmachung des Hinweises auf die Patenterteilung: **23.07.2025**
(21) Anmeldenummer: 21802214.3
(22) Anmeldetag: 21.10.2021
(51) Int. Cl.: A61B 17/70, A61B 17/86, B22F 5/06, B33Y 80/00

(54) **SCHRAUBENELEMENT OPTIMIERT FÜR DEN 3D-DRUCK**
SCREW ELEMENT OPTIMIZED FOR 3D PRINTING
ÉLÉMENT FILETÉ OPTIMISÉ POUR L'IMPRESSION 3D

(30) Priorität: 21.10.2020 DE 102020006464
(43) Veröffentlichungstag der Anmeldung: 30.08.2023
(73) Patentinhaber: Mimeo Medical GmbH, 70794 Filderstadt (DE)
(72) Erfinder: HEUER, Frank, 70794 Filderstadt (DE)
(74) Vertreter: Stolmár & Partner Patentanwälte PartG mbB
(86) Internationale Anmeldenummer: PCT/EP2021/079255
(87) Internationale Veröffentlichungsnummer: WO 2022/084464

(56) Entgegenhaltungen:
- DE-B3- 102014 208 012
- US-A1- 2011 060 373
- US-A1- 2012 197 311
- US-A1- 2015 196 336
- US-A1- 2017 202 585
- US-A1- 2017 348 033
- US-A1- 2019 239 935
- US-A1- 2019 343 564

## Beschreibung

### Stand der Technik

Dem Stand der Technik sind verschiedene Osteosynthesevorrichtungen wie zum Beispiel Schraubenelemente zur Fixation von Knochen oder Knochenfragmenten bekannt. Hergestellt werden solche Knochenschrauben klassisch mit CNC-Fräs- und Drehmaschinen. Für die besonderen Knochengewinde-Geometrien und vor allem auch für die unterschiedlichen Durchmesser der Schraubenelemente sind spezielle Gewindeplatten bereitzustellen, wenn sie in einer Serienfertigung hergestellt werden sollen. Dies führt zu längeren Lieferzeiten und höheren Kosten. Der 3D-Druck bietet eine mögliche Alternative hierzu, denn es können sämtliche Geometrien ohne Sonderwerkzeuge, und damit auch ohne Wartezeit auf Sonderwerkzeuge, hergestellt werden. Eine deutlich größere Flexibilität in der Geometrie-Gestaltung ist möglich.

Bei der Versorgung der Wirbelsäule, kommen beispielsweise Pedikelschrauben als Schraubenelemente zum Einsatz. Sie sind dadurch charakterisiert, dass sie zwei unterschiedliche Gewindebereiche besitzen. Distal ist ein Knochengewinde mit einer groben und proximal mit einer feineren Verzahnung vorgesehen. Eine grobe Verzahnung bietet besten Halt in spongiösem Knochen und eine feinere Verzahnung bietet höheren Halt an der Kortikalis. Solche Schraubenelemente, mit feiner und grober Verzahnung, gelten als der aktuelle Stand der Technik, da sie die besten Haltebedingungen an der Wirbelsäule mit einander kombinieren. Zwischen dem distalen und proximalen Gewinde befindet sich ein Gewindeübergangsbereich. In diesem Gewindeübergangsbereich wird von distal kommend zwischen den Gewindegängen des distalen Gewindes ein weiterer Gewindezahn vorgesehen, welcher sich dann in den proximalen Bereich erstreckt. Dadurch entsteht eine feinere Verzahnung im proximalen Bereich. Werden solche Schraubenelemente klassisch mit CNC-Dreh- und Fräsmaschinen hergestellt, ergibt sich fertigungsbedingt ein spiralförmiger Start des zusätzlichen Gewindeprofils, welcher entlang der Umfangsrichtung in radialer Richtung zunimmt. Beim Einschrauben der Pedikelschraube drückt sich dieser spiralförmige Gewindestart des Gewindeübergangsbereichs in den Knochen, um Platz für den nachfolgenden zusätzlichen Gewindegang zu schaffen. In einigen Fällen kann es passieren, insbesondere bei schwächeren Knochen, dass dieser raumfordernde Prozess des zusätzlichen Gewindegangs dazu führt, dass der Pedikel unbeabsichtigt gesprengt wird und eine Fraktur erzeugt wird. Dies ist auf die fehlende Gewinde-Vorschneidung zurückzuführen. Es wäre deshalb wünschenswert für den zusätzlichen Gewindegang eine Schneidkante vorzusehen. Dies ist mit herkömmlichen CNC-Fertigungsmethoden nur sehr schwer möglich. Der 3D-Druck bietet hierfür eine sehr gut Alternative.

Sollen Knochenschrauben mit dem 3D-Druck-Verfahren hergestellt werden, sind weitere Herausforderungen zu erwarten. Mit dem Blick auf die Kosteneffizienz in der Herstellung gilt es die Nachbehandlung der Teile nach dem 3D-Druck zu optimieren. Ein wesentlicher Optimierungsschritt ist die Reduktion sämtlicher für den Bau notwendigen Stützstrukturen, da sie aufwendig und oft manuell entfernt werden müssen. Ist die Baurichtung festgelegt, sollten keine geraden Flächen oder Überhänge in Baurichtung vorhanden sein, um genau diese Stützstrukturen einzusparen. Dies hat Implikationen auf Öffnungen und sonstigen Merkmale, wie zum Beispiel seitliche Fenestrationsöffnungen und auch die Werkzeugansatzstelle. Hier fehlen dem Stand der Technik entsprechende Merkmale, wie diese geometrisch vorgesehen sein müssen, um so wenig wie möglich Stützstrukturen verwenden zu müssen.

Ein Schraubenelement mit den Merkmalen des Oberbegriffs des Anspruchs 1 ist aus der US 2012/197311 A1 bekannt.

### Darstellung der Erfindung

Die erfindungsgemäße Lösung liegt in den Merkmalen des unabhängigen Anspruchs 1. Vorteilhafte Weiterbildungen der Erfindung sind Gegenstand der abhängigen Ansprüche.

Ein Verfahren zur Herstellung eines erfindungsgemäßen Schraubenelements wird ebenfalls beschrieben, das keinen Teil der beanspruchten Erfindung bildet. Es dient lediglich dem Verständnis der Erfindung.

Die herkömmliche CNC-Fertigung des Schraubenelements (1) mit einer Schneidkante am Gewindeübergangsbereich ist derzeit nicht oder nur unter höchsten technologischen Aufwand möglich. Deshalb stellt die additive Fertigung das Mittel der Wahl dar. Additive Fertigungen von metallischen Legierungen, oder auch 3D-Druck genannt, greifen auf das Laser- oder Elektronenstrahl-Schmelzverfahren zurück. Als Material eignen sich alle metallischen Legierungen, die als orthopädischer Implantatwerkstoff bekannt und akzeptiert sind. Dazu gehören beispielsweise Titan-, Cobalt-Chrom und Edelstahllegierungen.

Der Langzeit-Erfolg eines 3d-gedruckten Implantats ist stark von seiner Nachbehandlung abhängig. Eine gezielte Wärmebehandlung und Oberflächenbehandlung sind enorm wichtig. Hierzu ist einschlägige Literatur verfügbar, die die Zusammenhänge der Nachbehandlungen darlegen. Vorzugsweise werden die 3D-gedruckten Teile zuerst zwischen 500°C und maximal 850°C spannungsarm geglüht und anschließend einem Heiß-Isostatisches Press-Verfahren (HIP) ausgesetzt. Danach werden die Teile Korund-gestrahlt, um lose Partikel von der Oberfläche zu entfernen. Als weiterer Teil der Oberflächenbehandlung wird eine Glättung der Mikro-Strukturen durchgeführt. Hier kann mit Hilfe des chemischen Ätzens, welches optional durch eine galvanische Spannung und/oder durch mechanische Stimulation unterstützt werden kann, eine entsprechende Reduktion der Oberflächenrauigkeit erzielt werden. Ziel ist es, die unvollständig verschweißten Partikeln zu entfernen, da hier Zugspannungen und Mikro-Kerben auftreten, die durch die unvollständig-verschweißten Partikel entstehen, eine Schwächung der Dauerfestigkeit bedeuten können. Nach diesem Prozess eignet sich ein Kugelstrahlverfahren um Druckeigenspannungen an der Implantatoberfläche zu erzeugen. Dies steigert zusätzlich die Dauerfestigkeit.

Bei der Herstellung mit einem 3d-Druck-Verfahren sind einige Design-Parameter zu berücksichtigen. Einerseits muss eine Mindestwandstärke aller Strukturen von mindestens 0,1mm oder besser von noch mindestens 0,3mm eingehalten werden und andererseits müssen Spalte oder Schlitze mit einer Spaltdicke von mindestens 0,1mm, oder besser von mindestens 0,3mm aufweisen, damit beim additiven Herstellen, der Spalt auch offenbleibt und sich nicht unbeabsichtigt verschließt.

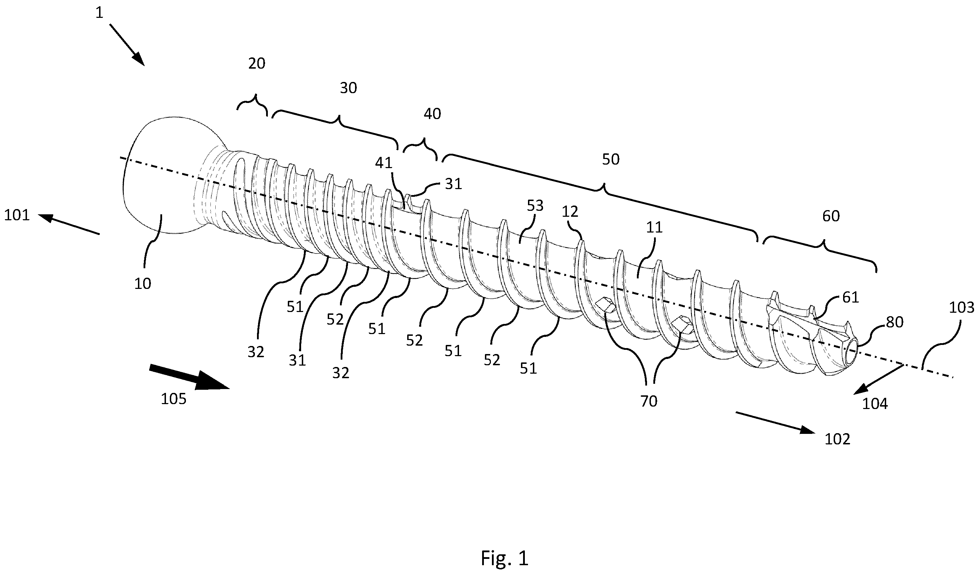

Für das erfindungsgemäße Schraubenelement (1), sind raumzuweisende Koordinatenreferenzen definiert, wie zum Beispiel die proximale Richtung (101), die distale Richtung (102), die sich entlang einer Zentralachse (103) ausdehnen. Von der Zentralachse (103) nach außen abgehend definiert sich die radiale Ausbreitung (104) (Fig. 1).

Zur Reduktion der Stützstrukturen beim 3D-Druck ist es vorteilhaft, wenn die Baurichtung (105) des Schraubenelements (1) in etwa der Richtung der Zentralachse (103) entspricht, und von proximal (101) nach distal (102) verläuft. Eine andere Orientierung würde verlangen, dass das Schraubenelement (1) schräg im 3D-Drucker aufgebaut werden müsste und eine Vielzahl von seitlichen Stützstrukturen vorgesehen sein müssten. Damit wäre die Herstellung weniger kosteneffizient. Optimalerweise wird der Kopfbereich (10) zuerst gefertigt. So muss nur der Kopf mit Stützstrukturen unterfüttert werden, und der Kopf (10) bietet mit seinem Durchmesser einen ausreichenden Seitenhalt, vor allem bei längeren Schraubenelementen. Mit einem größeren Stützendurchmesser müssen längere Bauteile beim 3D-Druck nicht zusätzlich in distaler Richtung gestützt werden.

Damit nicht die vollständige Fläche des Kopfbereichs (10) mit Stützstrukturen versehen sein muss, ist es vorteilhaft, wenn die Werkzeugansatzstelle (90) in proximaler Richtung (101) offen ist und in einer konzentrischen kegelartigen Ausnehmung (94) mündet und einen in etwa rechtwinkligen Konuswinkel besitz. Somit können die Stützstrukturen auf einen Stützstruktur-Ring (95) entlang des Durchmessers der Werkzeugansatzstelle reduziert werden.

Aus dem gleichen Grund ist es auch wichtig, dass die Werkzeugansatzstelle (90) nach distaler Richtung (102) von einer Wandung (93) begrenzt ist und diese Wandung (93) als Schräge in zunehmender distaler Richtung (102) nach radial innen verläuft und der durch die Wandung gebildete Konuswinkel kleiner als 120° ist. Vorzugsweise besitzt diese Wandung (93) einen in etwa rechtwinkligen Konuswinkel. Somit können auch hier Stützstrukturen für den Boden der Werkzeugansatzstelle eingespart werden. Das Entfernen etwaiger Stützstrukturen von dieser Bodenfläche (93) wäre eine große Herausforderung, da diese Bodenfläche (93) sehr schlecht für die Nachbearbeitung erreichbar ist.

In einer ersten Ausführungsform wird ein Schraubenelement (1) für die Fixation von Knochenkomponenten und Knochenfragmenten beschrieben, welcher aus einem Schaft (11), einem Halsbereich (20) und einen in proximaler Richtung (101) befindlichen Kopf (10), sowie in distaler Richtung (102) befindlicher Spitze (60) besteht. Der Kopf (10) ist vorzugsweise als Linse, Schrägkopf oder als Kugelkopf ausgebildet. Es sind aber auch eine Zusammensetzung aus unterschiedlichen Rundungen und Fläche denkbar. Hauptmerkmal des Kopfes ist es, dass der Kopf (10) einen größeren Außendurchmesser als der Halsbereich (20) aufweist. Vorzugsweise besitzt der Knochenanker eine Werkzeugansatzstelle (90), die zur Einleitung eines Drehmoments geeignet ist. Zur minimal-invasiven Versorgung ist es vorteilhaft, wenn der Knochenanker eine vollständig durchquerende Kanülierungsöffnung (80) besitzt, durch die ein chirurgischer Führungsdraht geführt werden kann.

Als Schraubenelement (1) kommen vorzugsweise Knochenschrauben zum Einsatz, die mit einem Knochen verschraubbar sind. Es sind aber auch Haken, Klemmen, Nägel und andersartig gestaltete Knochenanker anwendbar. In dem hier aufgeführten Beispiel eines Schraubenelements (1), wird eine Knochenschraube mit einem Schaft (11) und einen am Schaft befindlichen Knochengewinde (12) vorgestellt. Das Gewindegewinde (12) kann proximal zumindest abschnittsweise eine feinere Verzahnung (30) aufweisen, welches für einen härteren Kortikal-Knochen besser geeignet ist. Vorteilhaft ist ein nach distal spitz zulaufendes Gewinde (60) mit einer Schnittkante (61) an der Knochenankerspitze (60), damit sich der Schraubenelement (1) beim Einschrauben selbstschneidend in den Knochen ziehen kann.

Dabei ist es vorteilhaft wenn das Schraubenelement (1) dadurch charakterisiert ist, dass das Außengewinde (12) in einen an den Halsbereich (20) angrenzenden und in distaler Richtung (102) erstreckenden proximalen Gewindebereich (30), und ein sich daran angrenzender distaler Gewindebereich (50), und wiederrum daran angrenzender distaler Spitzenbereich (60) einteilen lässt, und der distale Gewindebereich (50) in den proximalen Gewindebereich (30) in einer Übergangszone (40) ineinander übergehen, und der proximale Gewindebereich (30) mindestens einen zusätzlichen Gewindegang (31, 32) ausbildet, welcher innerhalb der Übergangszone (40) mindestens eine Schneidkante (41) ausbildet.

Durch diese Schneidkante (41) wird eine vorschneidende Wirkung erzielt und keine Raumverdrängung im Knochen verursacht. Dies ist insbesondere bei schwächerem Knochen von klinischem Vorteil.

Weiterhin vorteilhaft ist, wenn wenigstens eine der Schneidkanten (41, 61) planar ist und hauptsächlich in radialer Richtung (104) ausgerichtet ist. Alternativ ist auch eine konkave oder konvexe Fläche zur Erzeugung der jeweiligen Schneidkante denkbar.

Als Knochengewinde sind unterschiedliche Gewindezahnverläufe und Anordnungen denkbar. Beispielsweise kann ein Gewinde mit einem Zahn am distalen Bereich auf ein Doppel- oder Dreifach-Gewinde in den proximalen Bereich übergehen. Es ist auch ein im distalen Bereich Doppel-Gewinde vorsehbar, welches in promxialer Richtung (101) in ein Vierfach- oder Sechsfach-Gewinde übergeht. Zur Vereinfachung sämtlicher Darstellungen wurde die bevorzugte Ausführungsform mit einem Doppelgewinde im distalen Bereich (50) und einem Vierfachgewinde am proximalen Bereich (30) abgebildet (Fig. 1-4).

Bei schwachem Knochen, wie beispielsweise bei einer Osteopenie oder Osteoporose, kann es notwendig sein, den Knochenanker zusätzlich zu augmentieren. Dies kann mit Knochenzement erfolgen. Knochenzement ist vorzugsweise ein Polymer, welches aus mindestens zwei Komponenten vermischt und im flüssigen bzw. pasten-ähnlichem Zustand injiziert wird. der Knochenzement härtet nach wenigen Minuten im Knochen zu einem Kunststoff aus und verbindet sich mit der schwamm-artigen Knochenstruktur. Angewandt wird meist ein Polymethylmethacrylat-Zement. Alternativ sind auch andere Medien für die Abgabe durch den Knochenanker denkbar. Es ist denkbar, dass alternative Medien, wie beispielsweise pharmazeutisch wirkende Medien, oder Medien mit Zellen, Nährstoffe, oder Medien die als Erbinformationsträger dienen, oder Impfstoffe durch den Knochenanker verabreicht werden.

Optional besitzt die Kanülierung (80) mindestens eine oder mehr seitlich abgehende und mit der Kanülierung kommunizierende Öffnungen (70). Vorzugsweise sind die Öffnungen in Umfangsrichtung in ring-artiger Formation (71 oder 72) angeordnet. Bei mehr als einer in Umfangsrichtung ring-artiger Öffnungs-Formation (71 und 72), haben die Öffnungen je Formation unterschiedliche Öffnungsquerschnittsflächen (710, 720). Bei in einen Knochen eingeschraubten Knochenanker (1), kommunizieren die seitlichen Öffnungen von der Hohlkammer (80) aus mit dem umgebenden Knochengewebe. Sie sind dazu geeignet, dass die Flüssigkeit, die in den Knochenanker (1) injiziert wird, durch die seitlichen Öffnungen in das umliegende Gewebe abgegeben wird. Eine unterschiedliche Querschittsfläche der Öffnungs-Formationen (710, 720) hat den Vorteil, dass aufgrund des lokalen Druckunterschieds innerhalb der Flüssigkeit, durch alle Öffnungen (71, 72) ein ähnlicher Volumenstrom generiert wird. Dies wird dadurch erreicht, dass die Öffnungen (72), die näher zur proximalen Richtung (101) liegen, eine kleinere Querschittsfläche (720) besitzen als die Öffnungen (710) der Formation (71), die weiter distal liegen.

Für den 3D-Druck besonders vorteilhaft ist, wenn diese seitlichen Öffnungen (70, 71, 72) als Polygon (700) vorgesehen sind. Herkömmlich werden solche seitlichen Öffnungen (70) konzentrisch ausgebohrt. Beim 3D-Druck würden konzentrische Öffnungen kleine Überhänge entstehen und daraus resultieren sogenannte Dross-Formationen (d.h. Miniatur-Tropfstein-ähnliche Formationen). Dies würde eine in der Serienfertigung kostspielige manuelle Nachbearbeitung erfordern. Eine Alternative, und damit bevorzugte Ausführungsform, bieten Polygone. Die schräg zulaufen Flächenelemente eines Polygons bilden einen Dachähnlichen Aufbau. Schrägen mit einem Winkel von etwa 45° sind problemlos im Druckverfahren ohne Stützstrukturen oder Dross-Formationen herzustellen.

Deshalb ist es vorteilhaft für die bevorzugte Ausführungsform, dass die seitlichen Öffnungen (70) als Polygon (700) vorgesehen sind. Dabei ist es vorteilhaft, wenn das Polygon (700) wenigstens ein Flächenelement (701, 702) besitzt, welches hauptsächlich entlang der Zentralachse (103) ausgeformt ist. Außerdem ist es optimal, wenn der Abstand zwischen den zur Zentralachse (103) parallel verlaufenden Flächenelementen (701 und 702) kleiner ist als der Kanülierungsdurchmesser D82 an der Mündungsstelle (83). Des Weiteren sollte das Polygon (700) wenigstens zwei Flächenelemente (z.B. 703, 704, 705, 706) besitzen, welche in einer Seitenansicht jeweils in einem Winkel von 25° bis 65°, vorzugsweise von 35° bis 55°, insbesondere von 40° bis 50° im Bezug zur Zentralachse (103) ausgerichtet sind, damit sie mit Hilfe eines 3D-Druckverfahrens entlang der definierten Baurichtung (105) überhaupt herstellbar sind.

In der bevorzugten Ausführungsform sind wenigstens zwei Flächenelemente (703, 704 oder 705, 706) symmetrisch im Bezug zur Zentralachse (103) zueinander orientiert. Alternative Ausgestaltungsformen sind ebenfalls denkbar, bei der das Polygon (700) beispielsweise als Rombus, Parallelogramm oder mit deutlich mehr Flächenelementen vorgesehen ist.

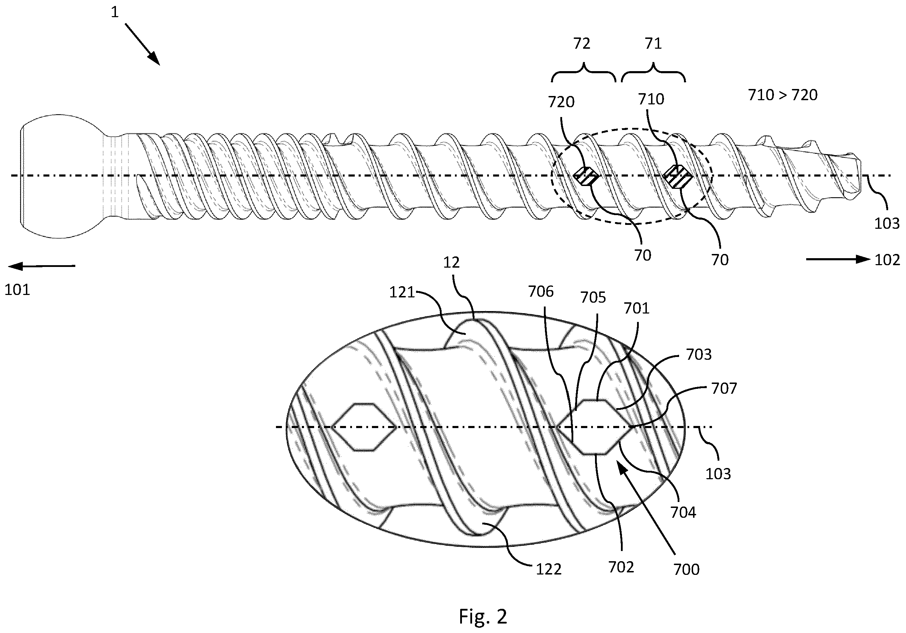

Mit dem 3D-Druck ist es auch möglich unterschiedlichen Rauigkeiten auf der Oberfläche des Schraubenelements (1) vorzusehen (Fig. 2). So ist es beispielsweise möglich auf den Oberflächen der Gewindeflanken (12), die nach proximaler Richtung (121) gerichtet sind, eine größere Oberflächenrauigkeit als auf den Oberflächen derselben Gewindeflanken, die nach distal gerichtet (122) sind. Eine höhere Rauigkeit auf den Gewindeflanken in proximaler Projektionsrichtung (121) haben den Vorteil, dass eine höhere Reibung zwischen Knochen und Schraubenelement in Auszugsrichtung erzeugt wird und das Schraubenelement eine signifikant höhere Auszugsfestigkeit erhält. Glattere Gewindeflanken in distaler Projektionsrichtung (122) haben den Vorteil, dass sich das Schraubenelement (1) dann trotzdem leicht in den Knochen einschrauben lässt.

Ein alternatives erfindungsgemäßes Schraubenelement (1) für die Fixation von Knochenkomponenten und Knochenfragmenten besteht aus einem Schaft (11) mit einem Außengewinde (12) und einer sich am Schaft (11) entlang erstreckenden longitudinalen Zentralachse (103), wodurch eine distale (102) und eine proximale (101) Richtung definiert wird. Das Schraubenelement (1) besitzt eine durchgängige Kanülierung (80). Die Kanülierung (80) besitzt mindestens zwei seitlich abgehende und mit der Kanülierung (80, 83) kommunizierende Öffnungen (70). Die Öffnungen (70) sind in einer Seitenansicht als Polygon (700) vorgesehen. Das Außengewinde (12) lässt sich in einen an den Halsbereich (20) angrenzenden und in distaler Richtung (102) erstreckenden proximalen Gewindebereich (30), und einen sich daran angrenzenden distalen Gewindebereich (50), und wiederrum daran angrenzenden distalen Spitzenbereich (60) einteilen. Der distale Gewindebereich (50) geht in den proximalen Gewindebereich (30) in einer Übergangszone (40) ineinander über. Der proximale Gewindebereich (30) bildet mindestens einen zusätzlichen Gewindegang (31, 32) aus, welcher innerhalb der Übergangszone (40) mindestens eine Schneidkante (41) ausbildet.

Ein weiteres alternatives erfindungsgemäßes Schraubenelement (1) für die Fixation von Knochenkomponenten und Knochenfragmenten besteht aus einem Schaft (11) mit einem Außengewinde (12) und einer sich am Schaft (11) entlang erstreckenden longitudinalen Zentralachse (103), wodurch eine distale (102) und eine proximale (101) Richtung definiert wird. Das Außengewinde (12) lässt sich in einen an den Halsbereich (20) angrenzenden und in distaler Richtung (102) erstreckenden proximalen Gewindebereich (30), und einen sich daran angrenzenden distalen Gewindebereich (50), und wiederrum daran angrenzender distaler Spitzenbereich (60) einteilen. Der distale Gewindebereich (50) geht in den proximalen Gewindebereich (30) in einer Übergangszone (40) ineinander über. Der proximale Gewindebereich (30) bildet mindestens einen zusätzlichen Gewindegang (31, 32) aus, welcher innerhalb der Übergangszone (40) mindestens eine Schneidkante (41) ausbildet.

### Kurze Beschreibung der Zeichnungen zeigen

Fig. 1 eine Schrägansicht des erfindungsgemäßen Schraubenelements,
Fig. 2 eine Seitensicht des erfindungsgemäßen Schraubenelements und eine Detaildarstellung der polygon-förmigen seitlichen Öffnungen.
Fig. 3 Seitenansicht und dazu gehöriger Schnittansicht durch den erfindungsgemäßen Knochenanker,
Fig. 4 zwei erfindungsgemäße Schraubenelemente im Verbund mit u-förmigen Gabelköpfen montiert mit einem Verbindungsstab.

### Beschreibung der bevorzugten Ausführungsformen

Fig. 1 zeigt das Schraubenelement (1) bestehend aus einen Kopfbereich (10), einen Halsbereich (20) und einen Schaft-Bereich (11) mit Knochengewinde (12). Weiterhin ist er erkennbar, dass das Außengewinde (12) in einen an den Halsbereich (20) angrenzenden und in distaler Richtung (102) erstreckenden proximalen Gewindebereich (30), und ein sich daran angrenzender distaler Gewindebereich (50), und wiederrum daran angrenzender distaler Spitzenbereich (60) einteilen lässt, und der distale Gewindebereich (50) in den proximalen Gewindebereich (30) in einer Übergangszone (40) ineinander übergeht, und der proximale Gewindebereich (30) mindestens einen zusätzlichen Gewindegang (31, 32) ausbildet, welcher innerhalb der Übergangszone (40) mindestens eine Schneidkante (41) ausbildet.

Eine weitere Schneidkante (61) ist an dem distalen Spitzenbereich (60) ausbildet. Optimalerweise verläuft die jeweilige Schneidkante (41, 61) hauptsächlich in radialer Richtung planar. Andere Flächengeometrien mit konvexen oder konkaven Bereichen sind ebenfalls denkbar. Alternativ sind auch in Umfangsrichtung wiederkehrende Muster, die eine vorschneidene Wirkung haben, wie beispielsweise Riffelungen oder Zähne, denkbar.

Fig. 1 illustriert in einer bevorzugten Ausführungsform ein Schraubenelement (1) welches im distalen Bereich (50) zwei voneinander getrennte Gewindezähne (51 und 52) ausbildet. Dies ist ein sogenanntes Doppelgewinde, wobei im Vergleich zu einem Einfachgewinde bei gleicher Gewindezahn-Anzahl eine größere Steigung erzielt wird. Das reduziert die notwendige Anzahl von Umdrehungen, um ein solches Schraubenelement (1) zu implantieren. Zwischen den Gewindegängen befindet sich der Gewindekern oder die Gewinde-Täler (53). Im proximalen Bereich (30) wird zwischen den distalen Gewindegängen (51, 52) jeweils ein zusätzlicher Gewindezahn (31, 32) vorgesehen. Die proximalen Gewindegänge (31, 32) besitzen dabei dieselbe Steigung, wie die distalen Gewindegänge (51 und 52). In dem Übergangsbereich (40) werden zwei Schneidkanten (41, 42) ausgebildet, wobei die zweite Schneidkante (42) aufgrund der Ansicht nicht darstellbar ist. Sie ist der (hier nicht sichtbare) Start für den zweiten proximalen Gewindegang (32). Die Schneidkanten (41, 42) haben den großen klinischen Vorteil, dass mit solchen Schraubenelementen (1) zukünftig Frakturen während der Implantation verhindert werden können. Ist alternativ ein anderes Gewinde vorgesehen, erhöhen oder reduzieren sich die Anzahl der Schneidkanten dementsprechend.

Der Fig. 1 ist außerdem zu entnehmen, dass die Baurichtung (105) des Schraubenelements (1) in etwa der Richtung der Zentralachse (103) entspricht, und von proximal (101) nach distal (102) verläuft. Die Vorteile wurden bereits eingangs beschrieben.

In Fig. 2 ist eine bevorzugte Ausführungsform eines Schraubenelements (1) dargestellt, bei dem die seitlichen Fenestrationsöffnungen (70) als Polygon (700) ausgestaltet sind. Das Polygon (700) besitzt wenigstens ein Flächenelement (701, 702), welches hauptsächlich entlang der Zentralachse (103) ausgeformt ist. Dabei ist der Abstand zwischen den zur Zentralachse (103) parallel verlaufenden Flächenelementen (701 und 702) kleiner ist als der Kanülierungsdurchmesser D82 an der Mündungsstelle (83). Des Weiteren besitzt das Polygon (700) wenigstens zwei Flächenelemente (z.B. 703, 704, 705, 706), welche in einer Seitenansicht jeweils in einem Winkel von 25° bis 65°, vorzugsweise von 35° bis 55°, insbesondere von 40° bis 50° im Bezug zur Zentralachse (103) ausgerichtet sind. Vorteilhaft ist es, wenn wenigstens zwei der Flächenelemente (703, 704 oder 705, 706) symmetrisch im Bezug zur Zentralachse (103) zueinander orientiert sind. Außerdem ist es denkbar, dass die Flächenelemente (701-706) mit Hilfe von Rundungen (707) ineinander übergehen.

In Fig. 2 außerdem darstellt ist, dass die seitlichen Öffnungen (70) in Umfangsrichtung in ring-artiger Formation (71 und/oder 72) angeordnet sind und bei mehr als einer in Umfangsrichtung ring-artiger Formation (71 und 72) die Öffnungen (70) je Formation unterschiedliche Öffnungsquerschnittsflächen (710, 720) aufweisen. Optimalerweise ist die Öffnungsquerschnittsfläche (710) der distalen Formation (71) größer als die Öffnungsquerschnittsfläche (720) der proximalen Formation (72).

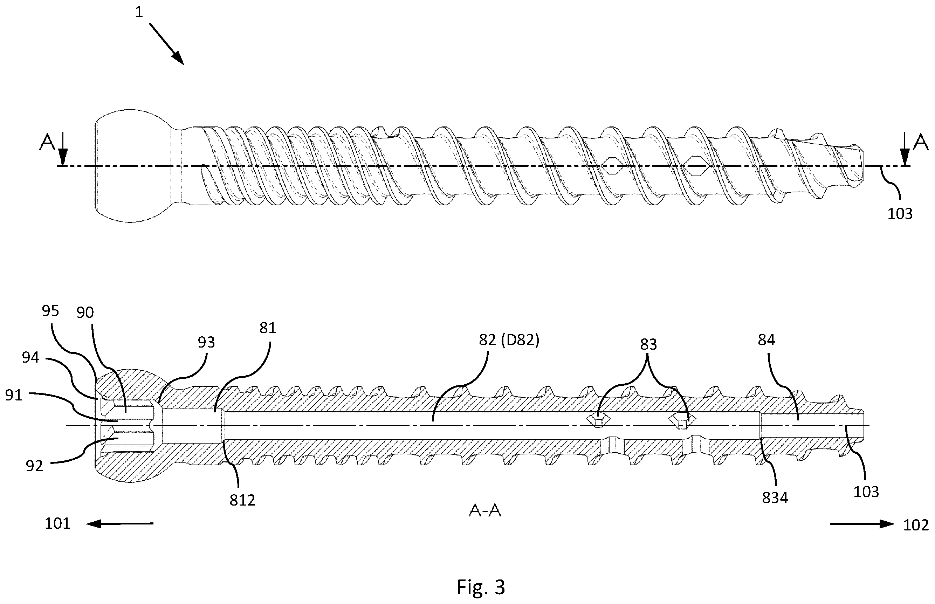

Fig. 3 offenbart eine Schnittansicht des Schraubenelements (1). Zu erkennen ist, das Innere des Kopfbereichs (10) und die durchgängige Kanülierung (80). Hauptmerkmal des Kopfes ist es, dass der Kopf (10) einen größeren Außendurchmesser als der Halsbereich (20) aufweist. Vorzugsweise besitzt der Knochenanker eine Werkzeugansatzstelle (90), welche zur Einleitung eines Drehmoments geeignet ist. Das Drehmoment zum Einschrauben des Knochenankers kam damit direkt über die Werkzeugansatzstelle eingeleitet werden. Diese Werkzeugansatzstelle kann ein beliebiges Profil (91,92) besitzen, wie beispielsweise einen Vielzahnrund, Innensechskant, Kreuzschlitz, einen einfachen Schlitz oder eine andersartig gestaltete Verzahnung aufweist. In der bevorzugten Ausführungsform befindet sich die Werkzeugansatzstelle (90) am proximalen Ende (101) und ist nach distaler Richtung (102) von einer Wandung (93) begrenzt. Diese Wandung (93) ist dabei als Schräge ausgeformt, die in zunehmender distaler Richtung (102) nach radial innen verläuft und der durch die Wandung gebildete Konuswinkel ist kleiner als 120°. Optimalerweise ist der Konuswinkel in etwa rechtwinklig. Des Weiteren ist erkennbar, dass die

Werkzeugansatzstelle (90) in proximaler Richtung (101) offen ist und in einer konzentrischen kegelartigen Ausnehmung (94) mündet und einen in etwa rechtwinkligen Konuswinkel besitzt. Der äußere proximale Ring fungiert dabei als eingangs beschriebener Stützstrukturen-Ring (95).

In Fig. 3 ebenfalls dargestellt, ist der Verlauf der Kanülierungsöffnung (80). Vorteilhaft ist es, wenn proximal ein Abschnitt (81) mit einem etwas größeren Durchmesser vorgesehen ist, in dem eine Applikationskanüle eingeführt werden kann. Daran angrenzend befindet sich der mittlere Teil der Kanülierung (82) mit einem Durchmesser D82. Die seitlichen Öffnungen (70) münden in die Kanülierung (82) durch entsprechende Mündungsstellen (83). Vorteilhaft ist es, wenn nach distal (102) der Kanülierungsdurchmesser in einem distalen Kanülierungsabschnitt (84) reduziert ist. Die Übergänge (812, 834) zwischen den unterschiedlichen Kanülierungsdurchmessern besitzen optimalerweise einen Konuswinkel von weniger als 120°, vorzugsweise sind sie in etwa rechtwinklig.

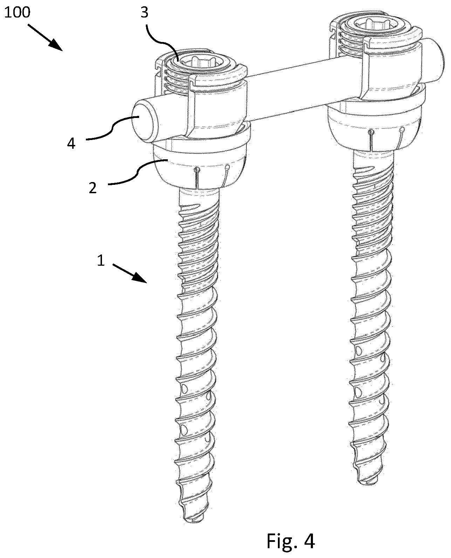

Fig. 4 illustriert zwei erfindungsgemäße Schraubenelemente (1) im Verbund mit u-förmigen Gabelköpfen (2) montiert mit einem Verbindungsstab (4). Die Schraubenelemente (1) besitzen einen proximalen Kopfbereich (10), welcher wenigstens abschnittsweise ein Kugelsegment aufweist, welches dazu geeignet ist, mit einen in einer Seitenansicht u-förmigen Gabelkopf (2), eine polyaxiale schwenkbare Verbindung bereitzustellen. Nach eingelegtem Verbindungsstab (4) und fixiertem Stellmittel (3) sind die Schraubenelemente (1) winkelstabil miteinander verbunden. Sie bilden eine rigide Fixierung, wie sie beispielsweise bei Wirbelsäuleneingriffen zum Einsatz kommt.

## Patentansprüche

1. Schraubenelement (1) für die Fixation von Knochenkomponenten und Knochenfragmenten bestehend aus einem Schaft (11) mit einem Außengewinde (12) und eine sich am Schaft (11) entlang erstreckende longitudinale Zentralachse (103) und dadurch eine distale (102) und eine proximale (101) Richtung definiert, und das Schraubenelement (1) eine durchgängige Kanülierung (80) besitzt, die Kanülierung (80) mindestens zwei seitlich abgehende und mit der Kanülierung (80, 83) kommunizierende Öffnungen (70) besitzt, wobei die Öffnungen (70) in einer Seitenansicht als Polygon (700) vorgesehen sind, **dadurch gekennzeichnet, dass** das Außengewinde (12) in einen an den Halsbereich (20) angrenzenden und in distaler Richtung (102) erstreckenden proximalen Gewindebereich (30), und ein sich daran angrenzender distaler Gewindebereich (50), und wiederrum daran angrenzender distaler Spitzenbereich (60) einteilen lässt, und der distale Gewindebereich (50) in den proximalen Gewindebereich (30) in einer Übergangszone (40) ineinander übergeht, und der proximale Gewindebereich (30) mindestens einen zusätzlichen Gewindegang (31, 32) ausbildet, welcher innerhalb der Übergangszone (40) mindestens eine Schneidkante (41) ausbildet.

2. Schraubenelement (1) nach vorhergehendem Anspruch, **dadurch gekennzeichnet, dass** das Polygon (700) wenigstens ein Flächenelement (701, 702) besitzt, welches hauptsächlich entlang der Zentralachse (103) ausgeformt ist.

3. Schraubenelement (1) nach einem der vorhergehenden Ansprüche, **dadurch gekennzeichnet, dass** der Abstand zwischen den zur Zentralachse (103) parallel verlaufenden Flächenelementen (701 und 702) kleiner ist als der Kanülierungsdurchmesser D82 an den Mündungsstellen (83) der Öffnungen (70).

4. Schraubenelement (1) nach einem der vorhergehenden Ansprüche, **dadurch gekennzeichnet, dass** das Polygon (700) wenigstens zwei Flächenelemente (z.B. 703, 704, 705, 706) besitzt, welche in einer Seitenansicht jeweils in einem Winkel von 25° bis 65°, vorzugsweise von 35° bis 55°, insbesondere von 40° bis 50° im Bezug zur Zentralachse (103) ausgerichtet sind.

5. Schraubenelement (1) nach einem der vorhergehenden Ansprüche, **dadurch gekennzeichnet, dass** wenigstens zwei Flächenelemente (703, 704 oder 705, 706) symmetrisch im Bezug zur Zentralachse (103) zueinander orientiert sind.

6. Schraubenelement (1) nach einem der vorhergehenden Ansprüche, **dadurch gekennzeichnet, dass** die Flächenelemente (701-706) mit Hilfe von Rundungen (707) ineinander übergehen.

7. Schraubenelement (1) nach einem der vorhergehenden Ansprüche, **dadurch gekennzeichnet, dass** die seitlichen Öffnungen (70) in Umfangsrichtung in ring-artiger Formation (71 und/oder 72) angeordnet sind und bei mehr als einer in Umfangsrichtung ring-artiger Formation (71 und 72) haben die Öffnungen (70) je Formation unterschiedliche Öffnungsquerschnittsflächen (710, 720).

8. Schraubenelement (1) nach Anspruch 7, **dadurch gekennzeichnet, dass** die Öffnungsquerschnittsfläche (710) der distalen Formation (71) größer ist als die Öffnungsquerschnittsfläche (720) der proximalen Formation (72).

9. Schraubenelement (1) nach einem der vorhergehenden Ansprüche, **dadurch gekennzeichnet, dass** die seitlichen Öffnungen (70) im Gewindegrund (53) platziert sind.

10. Schraubenelement (1) nach einem der vorhergehenden Ansprüche, **dadurch gekennzeichnet, dass** das Schraubenelement (1) zusätzlich einen Kopf (10), einen Halsbereich (20) und einen Schaft-Bereich (11) mit Knochengewinde (12) aufweist und eine Werkzeugansatzstelle (90) im Kopf (10) bereitgestellt wird und die Werkzeugansatzstelle (90) nach distaler Richtung (102) von einer Wandung (93) begrenzt ist und diese Wandung (93) als Schräge in zunehmender distaler Richtung (102) nach radial innen verläuft und der durch die Wandung gebildete Konuswinkel kleiner als 120° ist.

11. Schraubenelement (1) nach Anspruch 10, **dadurch gekennzeichnet, dass** die Wandung (93) einen in etwa rechtwinkligen Konuswinkel beschreibt.

12. Schraubenelement (1) nach den Ansprüchen 10 oder 11, **dadurch gekennzeichnet, dass** die Werkzeugansatzstelle (90) in proximaler Richtung (101) offen ist und in einer konzentrischen kegelartigen Ausnehmung (94) mündet und einen in etwa rechtwinkligen Konuswinkel besitzt.

13. Schraubenelement (1) nach einem der vorhergehenden Ansprüche, **dadurch gekennzeichnet, dass** der distaler Spitzenbereich (60) mindestens eine Schneidkante (61) ausbildet.

14. Schraubenelement (1) nach Anspruch 13, **dadurch gekennzeichnet**, wenigstens eine der Schneidkanten (41, 61) planar ist und hauptsächlich in radialer Richtung (104) ausgerichtet ist.

15. Schraubenelement (1) nach Anspruch 13, **dadurch gekennzeichnet**, wenigstens eine der Schneidkanten (41, 61) eine konkave Fläche besitzt, welche hauptsächlich in radialer Richtung (104) ausgerichtet ist.

16. Schraubenelement (1) nach Anspruch 13, **dadurch gekennzeichnet**, wenigstens eine der Schneidkanten (41, 61) eine konvexe Fläche besitzt, welche hauptsächlich in radialer Richtung (104) ausgerichtet ist.

## Claims

1. Screw element (1) for the fixation of bone components and bone fragments consisting of a shaft (11) with an external thread (12) and a longitudinal central axis (103) extending along the shaft (11) and thereby defining a distal (102) and a proximal (101) direction, and the screw element (1) has a continuous cannulation (80), the cannulation (80) has at least two laterally extending openings (70) communicating with the cannulation (80, 83), wherein the openings (70) are provided in a side view as a polygon (700), **characterized in that** the external thread (12) can be divided into a proximal thread region (30) adjoining the neck region (20) and extending in the distal direction (102), and a distal thread region (50) adjoining the proximal region, and in turn a distal tip region (60) adjoining the distal region, and the distal thread region (50) merges into the proximal thread region (30) in a transition zone (40), and the proximal thread region (30) forms at least one additional thread (31, 32), which forms at least one cutting edge (41) within the transition zone (40).

2. Screw element (1) according to the preceding claim, **characterized in that** the polygon (700) has at least one surface element (701, 702) which is formed mainly along the central axis (103).

3. Screw element (1) according to one of the preceding claims, **characterized in that** the distance between the surface elements (701 and 702) running parallel to the central axis (103) is smaller than the cannulation diameter D82 at the mouths (83) of the openings (70).

4. Screw element (1) according to one of the preceding claims, **characterized in that** the polygon (700) has at least two surface elements (e.g. 703, 704, 705, 706) which are in a side view each aligned at an angle of 25° to °, preferably of 35° to 55°, in particular of 40° to 50° with respect to the central axis (103).

5. Screw element (1) according to one of the preceding claims, **characterized in that** at least two surface elements (703, 704 or 705, 706) are oriented symmetrically with respect to one another in relation to the central axis (103).

6. Screw element (1) according to one of the preceding claims, **characterized in that** the surface elements (701-706) merge into one another by means of curves (707).

7. Screw element (1) according to one of the preceding claims, **characterized in that** the lateral openings (70) are arranged in a ring-like formation (71 and/or 72) in the circumferential direction and, in the case of more than one ring-like formation (71 and 72) in the circumferential direction, the openings (70) have different opening cross-sectional areas (710, 720) for each formation.

8. Screw element (1) according to claim 7, **characterized in that** the opening cross-sectional area (710) of the distal formation (71) is larger than the opening cross-sectional area (720) of the proximal formation (72).

9. Screw element (1) according to one of the preceding claims, **characterized in that** the lateral openings (70) are placed in the thread base (53).

10. Screw element (1) according to one of the preceding claims, **characterized in that** the screw element (1) furthermore has a head (10), a neck region (20) and a shaft region (11) with a bone thread (12) and a tool attachment point (90) is provided in the head (10) and the tool attachment point (90) is limited in the distal direction (102) by a wall (93) and this wall (93) extends radially inwards as a slope in the increasing distal direction (102) and the cone angle formed by the wall is smaller than 120°.

11. Screw element (1) according to claim 10, **characterized in that** the wall (93) describes an approximately right-angled cone angle.

12. Screw element (1) according to claims 10 or 11, **characterized in that** the tool attachment point (90) is open in the proximal direction (101) and opens into a concentric cone-like recess (94) and has an approximately right-angled cone angle.

13. Screw element (1) according to one of the preceding claims, **characterized in that** the distal tip region (60) forms at least one cutting edge (61).

14. Screw element (1) according to claim 13, **characterized in that** at least one of the cutting edges (41, 61) is planar and is mainly aligned in the radial direction (104).

15. Screw element (1) according to claim 13, **characterized in that** at least one of the cutting edges (41, 61) has a concave surface which is oriented mainly in the radial direction (104).

16. Screw element (1) according to claim 13, **characterized in that** at least one of the cutting edges (41, 61) has a convex surface which is oriented mainly in the radial direction (104) .

## Revendications

1. Élément fileté (1) pour fixer des composants osseux et des fragments osseux, constitué d'une tige (11) pourvue d'un filetage extérieur (12) et d'un axe central longitudinal (103) s'étendant le long de la tige (11) et définissant ainsi une direction distale (102) et une direction proximale (101), et l'élément fileté (1) possède une canulation continue (80), la canulation (80) possède au moins deux ouvertures (70) partant latéralement et communiquant avec la canulation (80, 83), les ouvertures (70) étant prévues sous forme de polygone (700) dans une vue latérale, **caractérisé en ce que** le filetage extérieur (12) peut être divisé en une zone de filetage proximale (30) adjacente à la zone de col (20) et s'étendant dans la direction distale (102), et une zone de filetage distale (50) adjacente à celle-ci, et à nouveau une zone de pointe distale (60) adjacente à celle-ci, et la zone de filetage distale (50) se raccorde à la zone de filetage proximale (30) dans une zone de transition (40), et la zone de filetage proximale (30) forme au moins un filet supplémentaire (31, 32) qui forme au moins une arête de coupe (41) à l'intérieur de la zone de transition (40).

2. Élément fileté (1) selon la revendication précédente, **caractérisé en ce que** le polygone (700) possède au moins un élément de surface (701, 702) qui est formé principalement le long de l'axe central (103).

3. Élément fileté (1) selon l'une des revendications précédentes, **caractérisé en ce que** la distance entre les éléments de surface (701 et 702) s'étendant parallèlement à l'axe central (103) est inférieure au diamètre de canulation D82 aux points d'embouchure (83) des ouvertures (70).

4. Élément fileté (1) selon l'une des revendications précédentes, **caractérisé en ce que** le polygone (700) possède au moins deux éléments de surface (par exemple 703, 704, 705, 706) qui, dans une vue latérale, sont orientés chacun selon un angle de 25° à 65°, de préférence de 35° à 55°, en particulier de 40° à 50° par rapport à l'axe central (103).

5. Élément fileté (1) selon l'une des revendications précédentes, **caractérisé en ce qu'**au moins deux éléments de surface (703, 704 ou 705, 706) sont orientés symétriquement l'un à l'autre par rapport à l'axe central (103).

6. Élément fileté (1) selon l'une des revendications précédentes, **caractérisé en ce que** les éléments de surface (701-706) se rejoignent à l'aide d'arrondis (707).

7. Élément fileté (1) selon l'une des revendications précédentes, **caractérisé en ce que** les ouvertures latérales (70) sont disposées en formation annulaire (71 et/ou 72) dans la direction circonférentielle et, dans le cas de plus d'une formation annulaire (71 et 72) dans la direction circonférentielle, les ouvertures (70) ont des surfaces de section d'ouverture différentes (710, 720) par formation.

8. Élément fileté (1) selon la revendication 7, **caractérisé en ce que** la surface de section d'ouverture (710) de la formation distale (71) est supérieure à la surface de section d'ouverture (720) de la formation proximale (72).

9. Élément fileté (1) selon l'une des revendications précédentes, **caractérisé en ce que** les ouvertures latérales (70) sont placées dans le fond de filet (53).

10. Élément fileté (1) selon l'une des revendications précédentes, **caractérisé en ce que** l'élément fileté (1) comprend en outre une tête (10), une zone de col (20) et une zone de tige (11) avec un filetage à os (12), et un point d'attache d'outil (90) est prévu dans la tête (10), et le point d'attache d'outil (90) est délimité dans la direction distale (102) par une paroi (93), et cette paroi (93) s'étend radialement vers l'intérieur sous la forme d'une pente dans une direction distale croissante (102), et l'angle conique formé par la paroi est inférieur à 120°.

11. Élément fileté (1) selon la revendication 10, **caractérisé en ce que** la paroi (93) décrit un angle conique à peu près à angle droit.

12. Élément fileté (1) selon les revendications 10 ou 11, **caractérisé en ce que** le point d'attache de l'outil (90) est ouvert dans la direction proximale (101) et débouche dans un évidement concentrique de type cône (94) et possède un angle cônique à peu près droit.

13. Élément fileté (1) selon l'une des revendications précédentes, **caractérisé en ce que** la zone de pointe distale (60) forme au moins une arête de coupe (61).

14. Élément fileté (1) selon la revendication 13, **caractérisé en ce qu'**au moins l'une des arêtes de coupe (41, 61) est plane et est orientée principalement dans la direction radiale (104).

15. Élément fileté (1) selon la revendication 13, **caractérisé en ce qu'**au moins l'une des arêtes de coupe (41, 61) possède une surface concave qui est orientée principalement dans la direction radiale (104).

16. Élément fileté (1) selon la revendication 13, **caractérisé en ce qu'**au moins l'une des arêtes de coupe (41, 61) possède une surface convexe qui est orientée principalement dans la direction radiale (104).
